(19) Europäisches Patentamt
European Patent Office
Office européen des brevets

(11) Publication number: 0 137 132
A2

(12) # EUROPEAN PATENT APPLICATION

(21) Application number: 84107875.1

(22) Date of filing: 05.07.84

(51) Int. Cl.⁴: C 07 H 7/00
C 07 H 17/08, A 61 K 31/00

(30) Priority: 18.07.83 YU 1553/83

(43) Date of publication of application: 17.04.85 Bulletin 85/16

(84) Designated Contracting States: AT BE CH DE FR GB IT LI SE

(71) Applicant: SOUR PLIVA farmaceutska, Kemijska prehrambena i kozmeticka industrija, n.sol.o.
Ive Lole Ribara 89
YU-41001 Zagreb(YU)

(72) Inventor: Lopotar, Nevenka M.Sc.
Munjarski put 2
YU-41000 Zagreb(YU)

(72) Inventor: Kobrehel, Gabrijela M.Sc.
A. Gramscia 1
YU-41000 Zagreb(YU)

(72) Inventor: Coric, Miljenko
Spasicev prilaz 3
YU-41000 Zagreb(YU)

(72) Inventor: Djokic, Slobodan D.Sc.
Pantovcak 59
YU-41000 Zagreb(YU)

(74) Representative: von Füner, Alexander, Dr. et al,
Patentanwälte v. Füner, Ebbinghaus, Finck Mariahilfplatz 2 & 3
D-8000 München 90(DE)

(54) **Process for the manufacture of 7,16-dioxa-2-aza-10-0-cladinosyl-12-0-desosaminyl-4,5-dihydroxy-6-ethyl-3,5,9,11,13,15-hexamethyl-bicyclo(11.2.1)-hexadeca-1(2)-ene-8-one and novel intermediate used in this process.**

(57) The invention relates to a new process for the manufacture of 7,16-dioxa-2-aza-10-0-cladinosyl-12-0-desosaminyl-4,5-dihydroxy-6-ethyl-3,5,9,11,13,15-hexamethyl-bicyclo[11.2.1]-hexadeca-1(2)-ene-8-one, a semisynthetic macrolide antibiotic of the erythromycin A series, by means of Beckmann rearrangement of erythromycin A oxime monohydrochloride or dihydrochloride with aromatic sulfochlorides of the formula 4-R-$C_6H_4SO_2Cl$, wherein R stands for $C_1$-$C_3$ alkyl, halogen, acylamino of the formula $-NHCOR_1$, wherein $R_1$ stands for $C_1$-$C_3$ alkyl, in the presence of bases, without preliminary isolation of erythromycin A oxime. There is also provided a novel imtermediate erythromycin A oxime dihydrochloride and the preparation thereof from erythromycin A with hydroxylamine hydrochloride.

EP 0 137 132 A2

Croydon Printing Company Ltd

Process for the manufacture of 7,16-dioxa-2-aza-10-O-cladinosyl-12-O-desosaminyl-4,5-dihydroxy-6-ethyl-3,5,9,11,13,15-hexamethylbicyclo[11.2.1]hexadeca-1(2)-ene-8-one and novel intermediate used in this process

This invention relates to a new process for the manufacture of 7,16-dioxa-2-aza-10-O-cladinosyl-12-O-desosaminyl-4,5-dihydroxy-6-ethyl-3,5,9,11,13,15-hexamethylbicyclo[11.2.1]hexadeca-1(2)-ene-8-one, a semisynthetic macrolide antibiotic of the erythromycin A series of the formula

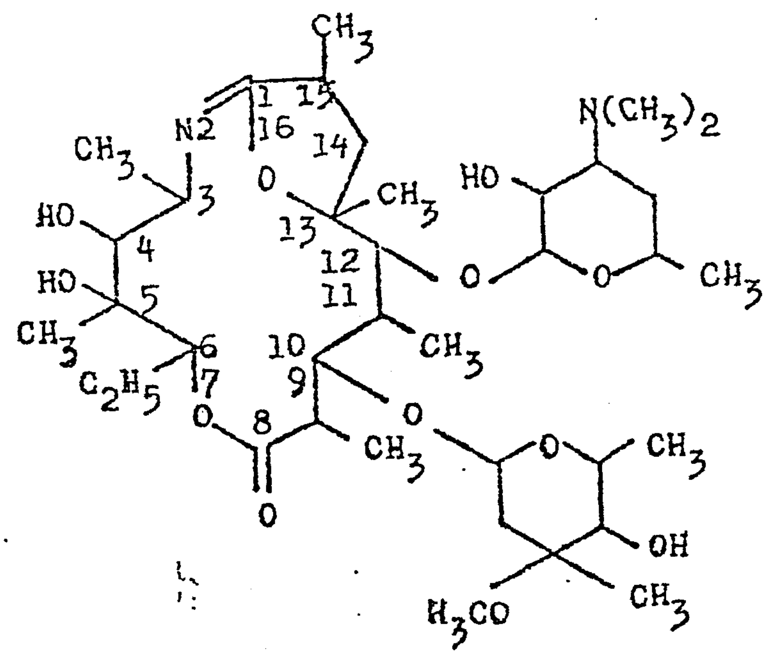

by means of Beckmann rearrangement of erythromycin A oxime mono- and dihydrochlorides with aromatic sulfochlorides in the presence of anorganic or organic bases and to the novel intermediate erythromycin A oxime dihydrochloride.

0137132

It is known that by oximation of erythromycin A with hydroxylamine hydrochloride in dry methanol in the presence of a large excess of barium carbonate at a pH value of about 7.0 there is obtained in a relatively small yield erythromycin A oxime monohydrochloride (m.p. 184-189°C; $R_f$ 0.25; sec. butanol-nitromethane-ethylacetate-water 6:3:2:2), which is easily converted by means of alcalization with NaOH into erythromycin A oxime (British patent 1,100,504; Tetrahedron Lett. 157, 1970).

It is also known that Beckmann rearrangement of erythromycin A oxime with aromatic sulfochlorides in the presence of anorganic or organic bases in a mixture of acetone and water yields 7,16-dioxa-2-aza-10-O-cladinosyl-12-O-desosaminyl-4,5-dihydroxy-6-ethyl-3,5,9,11,13,15-hexamethylbicyclo[11.2.1]-hexadeca-1(2)-ene-8-one (laid-open YU patent appln P 2764/82), which is an important intermediate in the synthesis of biologically active, semisynthetic erythromycins A with a widened 15-membered aglycone ring (U.S. patent 4,328,334, Belgian patent 892,357).

It has now been found that the reaction of erythromycin A with hydroxylamine hydrochloride in the presence of anorganic bases, such as $Na_2CO_3$ or $K_2CO_3$, in an inert solvent, preferably aliphatic alcohol such as methanol, at increased temperatures and at a pH value of the reaction mixture of 5.0 to 6.5 gives in a high yield the hitherto not described erythromycin A oxime dihydrochloride, which is by Beckmann rearrangement with aromatic sulfochlorides in the presence of anorganic or organic bases easily converted into 7,16-dioxa-2-aza-10-O-cladinosyl-12-O-desosaminyl-4,5-dihydroxy-6-ethyl-3,5,9,11,13,15-hexamethylbicyclo[11.2.1]hexadeca-1(2)-ene-8-one.

It has also been found that said product may be prepared by Beckmann rearrangement of erythromycin A oxime monohydrochloride at the above defined conditions.

According to this invention the oximation of erythromycin A is performed with a 1-5 molar excess of hydroxylamine hydrochloride and a 0-2 molar excess of anorganic bases such as sodium or

barium carbonates at increased temperatures, in an inert solvent such as an alcohol, at a pH of 5.0 to 6.5. After the completed reaction (10-22 hours) the reaction mixture is optionally filtered, concentrated by means of evaporation of the alcohol at reduced pressure to about 1/3 of the initial volume and left standing under cooling for 24 hours to crystallize. The filtration of the precipitated crystals and the washing of the precipitate with cold methanol yields erythromycin A oxime dihydrochloride (m.p. 140-145°C; $R_f$ 0.41; sec. butanol-nitromethane-etyhlacetate-water 6:3:2:2), which is without previous isolation of the erythromycin A oxime by means of Beckmann rearrangement with 1-4 moles of aromatic sulfochlorides of the formula $4\text{-R-}C_6H_4SO_2Cl$, wherein R stands for a lower alkyl radical of 1 to 3 carbon atoms, a halogen or an acylamino groups of the formula $-NHCOR_1$, wherein $R_1$ stands for a $C_1$-$C_3$ alkyl radical, and in the presence of 1-6 moles of an organic or anorganic base, such as $NaHCO_3$, NaOH or triethylamine, in a acetone-water medium (at a temperature from 0°C to reflux temperature of the mixture) easily converted into 7,16-dioxa-2-aza-10-O-cladinosyl-12-O-desosaminyl-4,5-dihydroxy-6-ethyl-3,5,9,11,13,15-hexamethylbicyclo[11.2.1]hexadeca-1(2)-ene-8-one. After the complated reaction (30 minutes to 24 hours) acetone is evaporated under reduced pressure and the product is isolated by a gradient pH extraction by means of methylene chloride, chloroform and any other non-polar solvent. In the same manner there is also performed Beckmann rearrangement of erythromycin A oxime monohydrochloride.

The invention is illustrated by the following Examples:

Example 1

Erythromycin A oxime dihydrochloride (Method A)

To a solution of erythromycin A (666 g, 0.907 mole) in methanol (3330 ml) there was added under stirring hydroxylamine hydrochloride (312 g, 4.489 mole) and sodium carbonate *sicc.* (79.5 g, 0.745 mole) and then kept at the boil for 10 hours under a reflux cooler. The hot reaction mixture was filtered (pH 5.8), the precipitate was repeatedly washed with hot methanol, and the combined methanol filtrates were concentrated to 1/3 of the initial volume by evaporation under reduced pressure. The reaction mixture was left standing for 24 hours to crystallize, the precipitated crystalline product was filtered, washed with cold methanol and dried at ambient temperature in a vacuum drier. There were obtained 570 g (76.4 %) of erythromycin A oxime dihydrochloride.

M.p. 140-145°C
$[\alpha]_D^{20}$ = -50.29° (1 %, $CH_3OH$)
IR (KBr): 1725 (C=O, lactone) and 1639 $cm^{-1}$ (C=N)
$^1H$ NMR (DMSO-d): 2.67 ppm (s, 6H, $-N(CH_3)_2$)
pK 7.3 (dimethylformamide-water, 66 %)
$R_f$ 0.41 (sec. butanol-nitromethane-ethylacetate-water 6:3:2:2)

| Analysis: | | calc. | found |
|---|---|---|---|
| | Cl | 8.63 % | 8.08 % |

Example 2

Erythromycin A oxime dihydrochloride (Method B)

From 666 g (0.907 mole) of erythromycin A, dissolved in 3330 ml of methanol, 312 g (4.489 mole) of hydroxylamine hydrochloride and 321 g (1.626 mole) of barium carbonate there was isolated, in accordance with the method described in Example 1, 563 g (75.5 %) of the product showing the same physical-chemical constants.

Example 3

7,16-dioxa-2-aza-10-O-cladinosyl-12-O-desosaminyl-4,5-dihydroxy-6-ethyl-3,5,9,11,13,15-hexamethylbicyclo[11.2.1]hexadeca-1(2)-ene-8-one (Method 1)

Solutions of p-toluene sulfochloride (2.32 g, 0.0122 mole) in acetone (20 ml) and $NaHCO_3$ (2.55 g, 0.0303 mole) in water (60 ml) were added dropwise during 30 minutes at a temperature of 0 to 5°C into a suspension of 5.0 g (0.00608 mole) of erythromycin A oxime dihydrochloride in acetone (50 ml). The reaction mixture was stirred for further 3 hours at the same temperature, the acetone was evaporated at reduced pressure and to the residual aqueous suspension there was added 10 ml of chloroform (pH 7.3) and then the pH was adjusted to 5.5 by acidification with 2 N HCl. The layers were separated and the aqueous layer was twice extracted with chloroform (10 ml). The extraction was repeated at pH 6.0 (3 x 10 ml) and pH 8.0 (3 x 20 ml) and the combined extracts were dried over $K_2CO_3$ and evaporated to dryness. At pH 8.0 there were isolated 3.3 g (74.3 %) of a chromatographically homogenous product of a $R_f$ 0.22 (n-butanol-acetic acid-water 4:1:5).

M.p. 128-131°C

IR ($CHCl_3$): 1725 (C=O, lactone) and 1705 $cm^{-1}$ (O-C=N)

$^{13}C$ NMR ($CDCl_3$): 178.1 (s, C-8), 163.9 (S, C-1) and 87.3 ppm (s, C-13)

$M^+$ 730

Example 4

7,16-dioxa-2-aza-10-O-cladinosyl-12-O-desosaminyl-4,5-dihydroxy-6-ethyl-3,5,9,11,13,15-hexamethylbicyclo[11.2.1]hexadeca-1(2)-ene-8-one (Method 2)

Solutions of p-toluene sulfochloride (2.42 g, 0.0127 mole) in acetone (20 ml) and $NaHCO_3$ (2.14 g, 0.0255 mole) in water (60 ml) were added dropwise during 1 hour at ambient temperature

0137132

to a suspension of 5.0 g (0.00636 mole) of erythromycin A oxime monohydrochloride in acetone (50 ml). The reaction mixture was stirred for two hours at the same temperature, the acetone was evaporated under reduced pressure and the product was isolated by gradient pH extraction with chloroform at a pH of 5.5 (3 x 10 ml), pH 6.0 (3 x 10 ml) and pH 8.0 (3 x 20 ml). After the drying of the combined organic extracts over $K_2CO_3$ there was isolated at a pH of 8.0 2.12 g (45.6 %) of the product showing the same physical-chemical constants as described in Example 3.

Example 5

7,16-dioxa-2-aza-10-O-cladinosyl-12-O-desosaminyl-4,5-dihydroxy-6-ethyl-3,5,9,11,13,15-hexamethylbicyclo[11.2.1]hexadeca-1(2)-ene-8-one (Method 3)

Into a suspension of erythromycin A oxime dihydrochloride (5.0 g, 0.00608 mole) in acetone (50 ml) there was added triethyl amine (3.074 g, 0.03074 mole) and then there was added dropwise under stirring at a temperature of 0 to 5°C during 30 minutes a solution of p-toluene sulfochloride (2.32 g, 0.01216 mole) in acetone (20 ml). The reaction mixture was stirred for two hours at the same temperature and then left standing for 24 hours at ambient temperature. The acetone was evaporated at reduced pressure, the residual precipitate was suspended in 50 ml of water and 10 ml of chloroform (pH 7.7) and extracted by means of a gradient pH extraction with chloroform at pH 5.5 (3 x 10 ml) and at pH 8.0 (3 x 20 ml). After drying the combined organic extracts over $K_2CO_3$ and the evaporation of chloroform there was obtained at pH 8.0 2.94 g (66.1 %) of the product of the same physical-chemical constants as described in Example 3.

Example 6

7,16-dioxa-2-aza-10-O-cladinosyl-12-O-desosaminyl-4,5-dihydroxy-6-ethyl-3,5,9,11,13,15-hexamethylbicyclo[11.2.1]hexadeca-1(2)-ene-8-one (Method 4)

0137132

To a suspension of erythromycin A oxime monohydrochloride (5.0 g, 0.00636 mole) in acetone (50 ml) there were added 2.574 g (0.0257 mole) of triethylamine, and then there was added dropwise under stirring at 0 to 5°C 2.425 g (0.0127 mole) of p-toluene sulfochloride in 20 ml of acetone. In accordance with the process described in Example 5, there were isolated at pH 8.0 3.3 g (70.9 %) of a product of the same physical-chemical constants as described in Example 3.

Example 7

7,16-dioxa-2-aza-10-O-cladinosyl-12-O-desosaminyl-4,5-dihydroxy-6-ethyl-3,5,9,11,13,15-hexamethylbicyclo[11.2.1]hexadeca-1(2)-ene-8-one (Method 5)

Starting from 5.0 g (0.00608 mole) of erythromycin A oxime dihydrochloride suspended in acetone (50 ml), 7.35 g (0.0243 mole) of p-iodobenzene sulfochloride dissolved in 70 ml of acetone and 10.22 g (0.122 mole) of $NaHCO_3$ dissolved in 210 ml of water, there were obtained and isolated in accordance with the process described in Example 3 2.98 g (66.9 %) of the product of the same physical-chemical constants as described in Example 3.

Example 8

7,16-dioxa-2-aza-10-O-cladinosyl-12-O-desosaminyl-4,5-dihydroxy-6-ethyl-3,5,9,11,13,15-hexamethylbicyclo[11.2.1]hexadeca-1(2)-ene-8-one (Method 6)

Starting from 5.0 g (0.00608 mole) of erythromycin A oxime dihydrochloride, suspended in 50 ml of acetone, 2.84 g (0.0122 mde) of p-acetylaminobenzene sulfochloride,dissolved in 180 ml of an acetone-water mixture (1:1),and 2.55 g (0.0304 mole) of $NaHCO_3$, dissolved in 90 ml of water, there was obtained and isolated in accordance with the process described in Example 3, 2.73 g (61.4 %) of the product of the same physical-chemical constants as described in Example 3.

Example 9

7,16-dioxa-2-aza-10-O-cladinosyl-12-O-desosaminyl-4,5-dihydroxy-6-ethyl-3,5,9,11,13,15-hexamethylbicyclo[11.2.1]hexadeca-1(2)-ene-8-one (Method 7)

To a solution of erythromycin A (55.5 g, 0.076 mole) in methanol (280 ml) there were added under stirring hydroxylamine hydrochloride (26.01 g, 0.373 mole) and sodium carbonate sicc. (6.62 g, 0.062 mole), whereupon it was kept at the boil for 18 hours under a reflux cooler. The methanol was distilled off under reduced pressure (about 2/3 of the volume) and the concentrated reaction suspension was left to crystallize for 24 hours at 0 to 8°C, filtered and washed with cold methanol. The obtained crystalline product (52.7 g) was suspended in acetone (525 ml), the reaction suspension was cooled to a temperature of 0 to 5°C and thereupon there were added dropwise under stirring p-toluenesulfochloride (24.4 g, 0.128 mole), dissolved in acetone (210 ml), and $NaHCO_3$ (21.5 g, 0.526 mole), dissolved in water (630 ml). The reaction mixture was stirred for further 3 hours at the same temperature, the acetone was evaporated and the product was isolated by a gradient pH extraction as described in Example 3. The yield was 34.7 g (62.1 %) of the product showing the same physical-chemical constants as described in Example 3.

Example 10

7,16-dioxa-2-aza-10-O-cladinosyl-12-O-desosaminyl-4,5-dihydroxy-6-ethyl-3,5,9,11,13,15-hexamethylbicyclo[11.2.1]hexadeca-1(2)-ene-8-one (Method 8)

Erythromycin A oxime dihydrochloride (30.7 g), obtained in accordance with the process described in Example 9, was suspended in a mixture of acetone (470 ml) and water (200 ml), cooled to 0 to 5°C, whereupon there was added to the obtained suspension under stirring during 1 hour step by step solid

p-acetylaminobenzene sulfochloride (17.3 g, 0.07403 mole) and $NaHCO_3$ (15.6 g, 0.186 mole), dissolved in water (230 ml). The reaction mixture was stirred for 3 hours at the same temperature, the acetone was evaporated and the product was isolated as described in Example 3. Yield: 16.0 g (56.01 %).

WHAT IS CLAIMED IS:

1. Erythromycin A oxime dihydrochloride.

2. Process for the manufacture of 7,16-dioxa-2-aza-10-O-cladinosyl-12-O-desosaminyl-4,5-dihydroxy-6-ethyl-3,5,9,11,13,15-hexamethylbicyclo[11.2.1]hexadeca-1(2)-ene-8-one of the formula

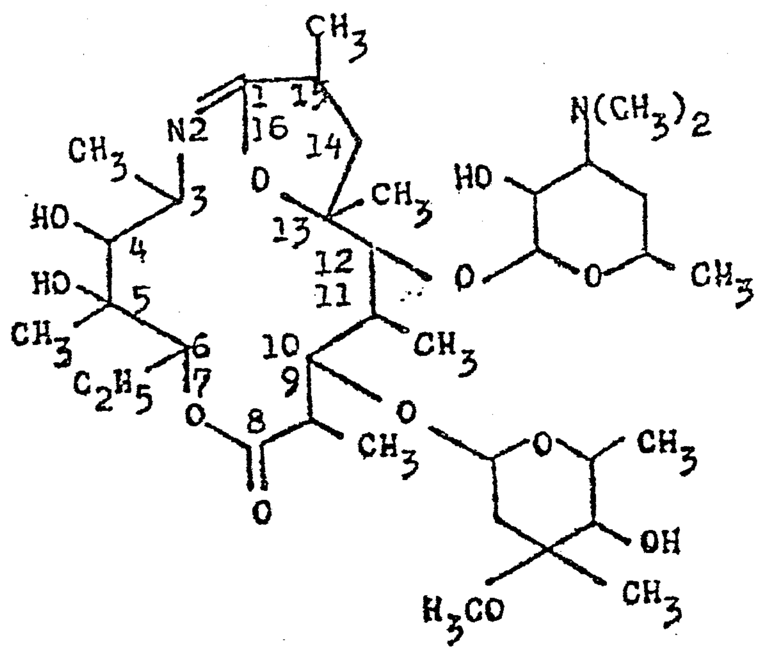

characterized in that erythromycin A oxime monohydrochloride or erythromycin A oxime dihydrochloride, which is obtained by oximation of erythromycin A with a 1-5 molar excess of hydroxylamine hydrochloride in the presence of a 0-2 molar excess of of an anorganic base, such as sodium or barium carbonate, in an alcohol at increased temperature and at a pH of the reaction mixture of 5.0 to 6.5,
are subjected to Beckmann rearrangement with 1-4 moles of aromatic sulfochlorides of the formula

$$4\text{-}R\text{-}C_6H_4SO_2Cl$$

wherein R stand for a $C_1$-$C_3$ alkyl, a halogen, or an acylamino groups of the formula $-NHCOR_1$, wherein $R_1$ stands for a $C_1$-$C_3$

alkyl, in the presence of anorganic or organic bases such as $NaHCO_3$, NaOH or triethylamine at a temperature of $0^oC$ to reflux temperature, in a mixture of acetone and water.